# EUROPÄISCHE PATENTSCHRIFT

(11) **EP 1 003 725 B1**
(45) Veröffentlichungstag und Bekanntmachung des Hinweises auf die Patenterteilung: **29.12.2004**
(21) Anmeldenummer: 98942623.4
(22) Anmeldetag: 25.07.1998
(51) Int. Cl.: C07D 231/16, A01N 43/48

(54) **FLUORPYRAZOL-BIPHENYLAMIDE ALS FUNGIZIDE**
FLUOROPYRAZOLE-BIPHENYLAMIDE FUNGICIDES
FLUORPYRAZOLE-BIPHENYLAMIDES FONGICIDES

(30) Priorität: 15.08.1997 DE 19735224
(43) Veröffentlichungstag der Anmeldung: 31.05.2000
(73) Patentinhaber: BASF AKTIENGESELLSCHAFT, 67056 Ludwigshafen (DE)
(72) Erfinder: EICKEN, Karl, D-67157 Wachenheim (DE); RACK, Michael, D-69123 Heidelberg (DE); WETTERICH, Frank, D-67112 Mutterstadt (DE); AMMERMANN, Eberhard, D-64646 Heppenheim (DE); LORENZ, Gisela, D-67434 Neustadt (DE); STRATHMANN, Siegfried, D-67117 Limburgerhof (DE)
(74) Vertreter: Pohl, Michael, Dr.
(86) Internationale Anmeldenummer: PCT/EP1998/004663
(87) Internationale Veröffentlichungsnummer: WO 1999/009013

(56) Entgegenhaltungen:
- EP-A- 0 276 177
- EP-A- 0 545 099
- EP-A- 0 589 301
- EP-A- 0 776 889
- WO-A-96/38419
- DE-A- 2 417 216
- PATENT ABSTRACTS OF JAPAN vol. 12, no. 263 (C-514), 22. Juli 1988 & JP 63 048269 A (SUMITOMO CHEM CO LTD), 29. Februar 1988

## Beschreibung

Die vorliegende Erfindung betrifft Biphenylamide der allgemeinen Formel I 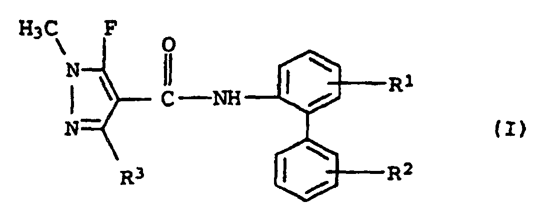 sowie deren Salze, in der die Reste R¹, R² und R³ die folgenden Bedeutungen haben:
- R¹: Wasserstoff oder Fluor;
- R²: Wasserstoff, Halogen, C₁-C₄-Alkyl, Halogenmethyl, insbesondere Fluor- oder Chlormethyl, wie z.B. Trifluormethyl, Trichlormethyl, C₁-C₄-Alkoxy oder C₁-C₄-Alkylthio;
- R³: Methyl, Difluormethyl oder Trifluormethyl.

Außerdem betrifft die Erfindung ein Verfahren zur Herstellung der Verbindungen I, I enthaltende Mittel sowie ein Verfahren zur Bekämpfung von Schadpilzen und die Verwendung der Verbindungen I, ihrer Salze oder der Mittel hierzu.

Fungizide Biphenylamide des Typs I sind aus folgenden Druckschriften bekannt: US 5 438 070, DE-A 24 17 216, EP-A 545 099 und EP-A 589 301. Die dort genannten Wirkstoffe können jedoch hinsichtlich ihrer Wirkung noch nicht befriedigen.

EP 776 889 beschreibt fungizide Fluorpyrazole. JP 63-048 269 betrifft Pyrazolcarboxamidverbindungen und diese als aktive Komponente enthaltende Fungizide.

Der vorliegenden Erfindung lagen daher Biphenylamide mit besserer Wirkung gegen Schadpilze als Aufgabe zugrunde.

Demgemäß wurden die eingangs definierten Verbindungen I gefunden.

Ferner wurden Mittel, welche die Verbindungen I oder deren Salze enthalten sowie ein Verfahren zur Herstellung von I und der Mittel gefunden. Des weiteren wurden ein Verfahren zur Bekämpfung von Schadpilzen sowie die Verwendung der Verbindungen I, ihrer Salze oder der Mittel hierzu gefunden.

Die Verbindungen I sind in an sich bekannter Weise aus den entsprechenden Carbonsäurehalogeniden II und den Biphenylaminen III unter Zuhilfenahme einer Base, wie z.B. Triethanolamin, erhältlich. 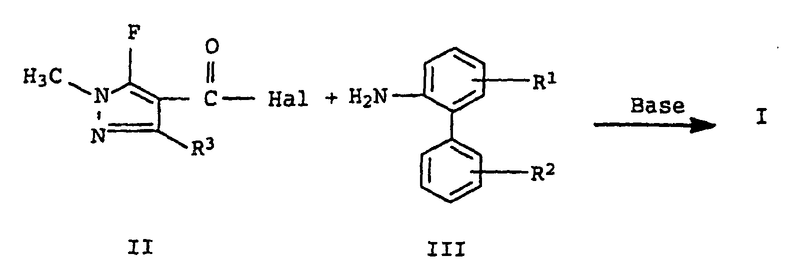
- Hal: Halogen, vorzugsweise Chlor oder Brom;
- R¹: Wasserstoff oder Fluor;
- R²: Wasserstoff, Halogen, C₁-C₄-Alkyl, Halogenmethyl, insbesondere Fluor- oder Chlormethyl, wie z.B. Trifluormethyl, Trichlormethyl, C₁-C₄-Alkoxy oder C₁-C₄-Alkylthio;
- R³: Methyl, Difluormethyl oder Trifluormethyl

Hinsichtlich der Reaktionsbedingungen für die Herstellung der Verbindungen I und was die Herkunft der Ausgangsverbindungen II angeht vgl. z.B. WO 93/11117, Seite 17-19.

Die Biphenylamine III sind allgemein bekannt oder in an sich bekannter Weise erhältlich (vgl. z.B. Tetrahedron Letters 28, Seite 5093 bis Seite 5096, 1987).

Teil der Erfindung sind auch die Salze der säurebeständigen Verbindungen I, welche basische Zentren, vor allem basische Stickstoffatome enthalten, insbesondere mit Mineralsäuren wie Schwefelsäure und Phosphorsäure oder Lewis-Säuren wie Zinkchlorid. Üblicherweise kommt es hierbei auf die Art des Salzes nicht an. Im Sinne der Erfindung sind solche Salze bevorzugt, die die von Schadpilzen freizuhaltenden Pflanzen, Flächen, Materialien oder Räume nicht schädigen und die Wirkung der Verbindungen I nicht beeinträchtigen. Besonders bedeutsam sind derartige, für landwirtschaftliche Zwecke geeignete Salze.

Die Salze der Verbindungen I sind in an sich bekannter Weise zugänglich, vor allem durch Umsetzen der entsprechenden Biphenylamide I mit den genannten Säuren in Wasser oder einem inerten organischen Lösungsmittel bei Temperaturen von -80 bis 120°C, vorzugsweise 0 bis 60°C.

Bei den eingangs angegebenen Definitionen der Verbindungen I bis III wurden Sammelbegriffe verwendet, die repräsentativ für die folgenden Substituenten stehen:
Halogen: Fluor, Chlor, Brom und Jod;
Alkyl: geradkettige oder verzweigte Alkylgruppen mit 1 bis 4 Kohlenstoffatomen, z.B. C₁-C₄-Alkyl wie Methyl, Ethyl, n-Propyl, 1-Methylethyl, n-Butyl, 1-Methylpropyl, 2-Methylpropyl, 1,1-Dimethylethyl, 1-Methylbutyl, 2-Methylbutyl, 3-Methylbutyl;
Alkoxy: geradkettige oder verzweigte Alkoxygruppen mit 1 bis 4 Kohlenstoffatomen, z.B. C₁-C₃-Alkoxy wie Methyloxy, Ethyloxy, Propyloxy und 1-Methylethyloxy;
Alkylthio: geradkettige oder verzweigte Alkylgruppen mit 1 bis 4 Kohlenstoffatomen (wie vorstehend genannt), welche über ein Schwefelatom (-S-) an das Gerüst gebunden sind, z.B. Methylthio, Ethylthio, Propylthio, 1-Methylethylthio, n-Butylthio und tert.-Butylthio.

Im Hinblick auf ihre biologische Wirkung gegen Schadpilze sind Verbindungen I bevorzugt, in denen R² steht für
- Halogen, vor allem Fluor, Chlor oder Brom;
- C₁-C₄-Alkyl, vor allem Methyl, Ethyl, Propyl, 1-Methylethyl, Butyl, 1-Methylpropyl, 2-Methylpropyl oder 1,1-Dimethylethyl;
- C₁-C₄-Alkoxy, vor allem Methoxy, Ethoxy, Propoxy, 1-Methylethoxy, Butoxy, 1-Methylpropoxy, 2-Methylpropoxy oder 1,1-Dimethylethoxy;
- Alkylthio, vor allem Methylthio, Ethylthio oder n- oder iso-Propylthio.

Ganz besonders bevorzugt sind im Hinblick auf ihre Verwendung zur Bekämpfung von Schadpilzen die in der folgenden Tabelle 1 zusammengestellten Verbindungen I.

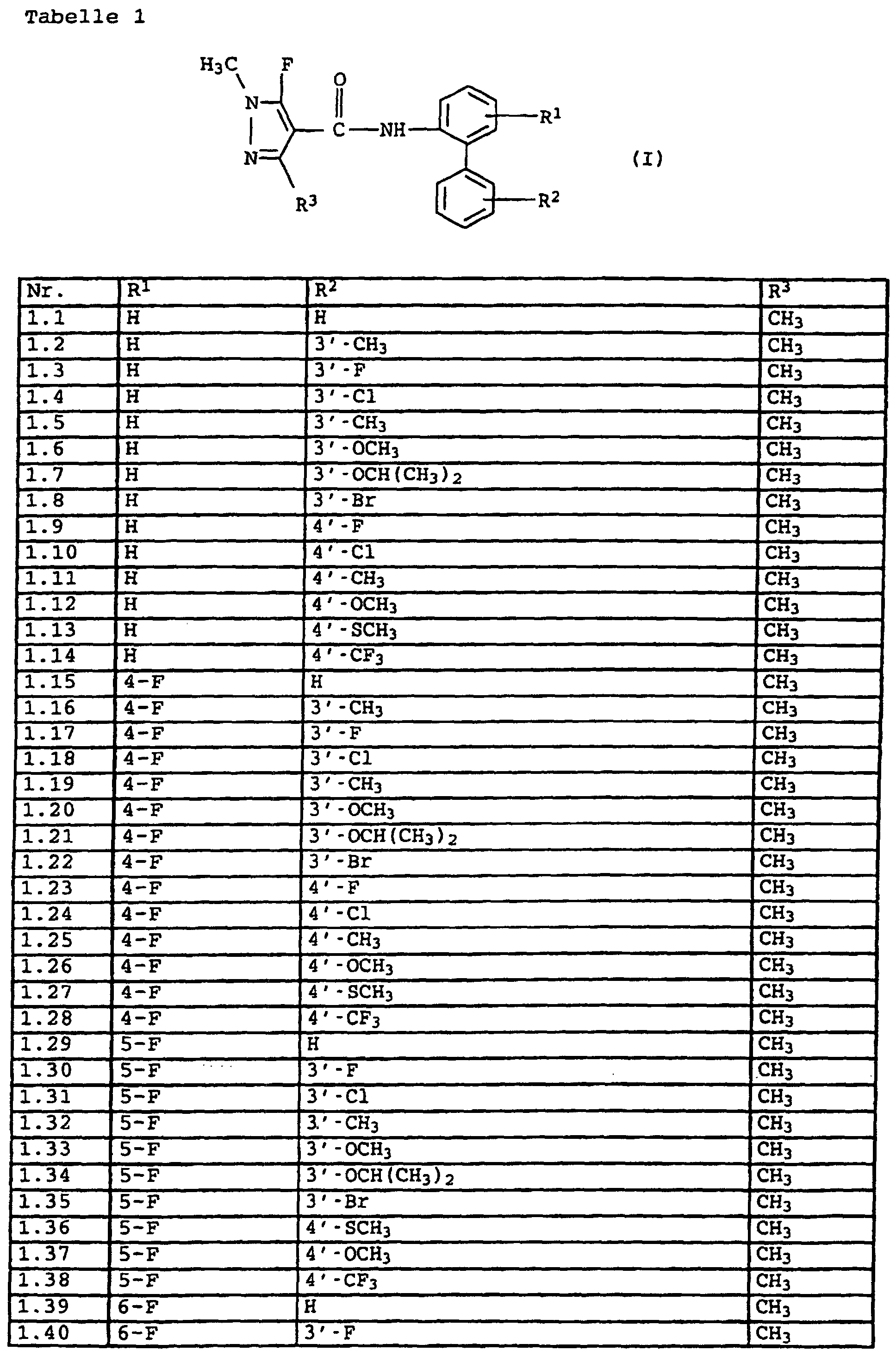

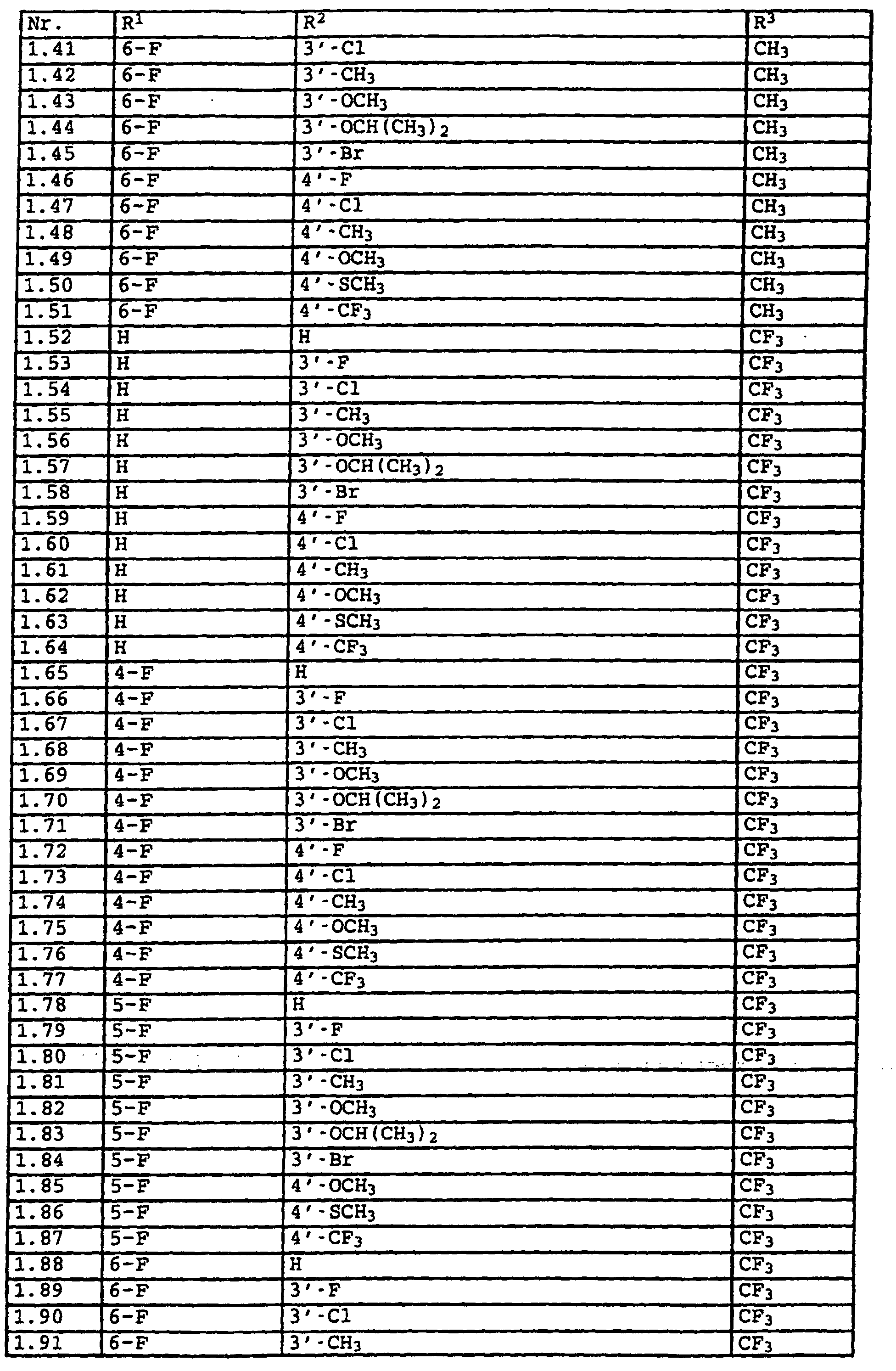

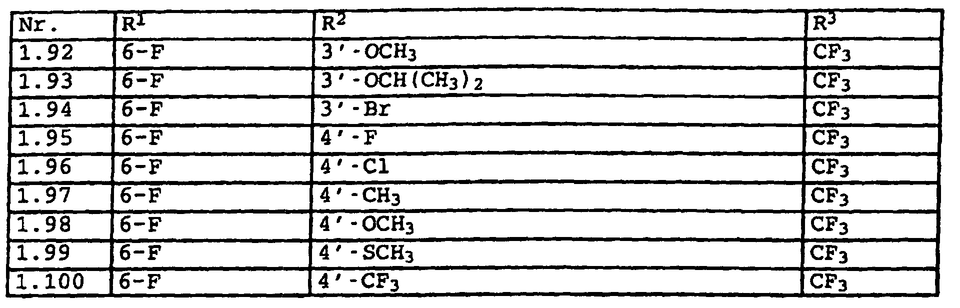

Die fungizid wirkenden Verbindungen I und ihre Salze können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden unter Verwendung üblicher Formulierungshilfsmittel - wie im folgenden beschrieben - und in an sich bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Lauryletherund Fettalkoholsulfaten, sowie Salze sulfatierter Hexa-, Heptaund Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes iso-Octyl-, Octyl- oder Nonylphenol, Alkylphenol-, Tributylphenylpolyglykolether, Alkylarylpolyetheralkohole, iso-Tridecylalkohol, Fettalkoholethylenoxid-Kondensate, ethoxyliertes Rizinusöl, Polyoxyethylenalkyloder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:
I. eine Lösung aus 90 Gew.-Teilen einer erfindungsgemäßen Verbindung I und 10 Gew.-Teilen N-Methyl-2-pyrrolidon, die zur Anwendung in Form kleinster Tropfen geeignet ist;
II. eine Mischung aus 10 Gew.-Teilen einer erfindungsgemäßen Verbindung I, 70 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure, 5 Gew.-Teilen des Anlagerungsproduktes von 40 Mol Ethylenoxid an 1 Mol Ricinusöl; durch feines Verteilen der Lösung in Wasser erhält man eine Dispersion.
III. eine wäßrige Dispersion aus 10 Gew.-Teilen einer erfindungsgemäßen Verbindung I, 40 Gew.-Teilen Cyclohexanon, 30 Gew.-Teilen iso-Butanol, 20 Gew.-Teilen des Anlagerungsproduktes von 40 mol Ethylenoxid an 1 mol Ricinusöl;
IV. eine wäßrige Dispersion aus 10 Gew.-Teilen einer erfindungsgemäßen Verbindung I, 25 Gew.-Teilen Cyclohexanol, 55 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;
V. eine in einer Hammermühle vermahlene Mischung aus 80 Gew.-Teilen, vorzugsweise einer festen erfindungsgemäßen Verbindung I, 3 Gew.-Teilen des Natriumsalzes der Di-isobutylnaphthalin-2-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;
VI. eine innige Mischung aus 3 Gew.-Teilen einer erfindungsgemäßen Verbindung I und 97 Gew.-Teilen feinteiligem Kaolin; dieses Stäubemittel enthält 3 Gew.-% Wirkstoff;
VII. eine innige Mischung aus 30 Gew.-Teilen einer erfindungsgemäßen Verbindung I, 62 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen einer erfindungsgemäßen Verbindung I, 10 Gew.-Teilen des Natriumsalzes eines Phenolsulfonsäure-Harnstoff-Formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen einer erfindungsgemäßen Verbindung I, 2 Gew.-Teilen des Calciumsalzes der Dodecylbenzolsulfonsäure, 8 Gew.-Teilen Fettalkoholpolyglykolether, 20 Gew.-Teilen des Natriumsalzes eines Phenolsulfonsäure-Harnstoff-Formaldehyd-Kondensates und 50 Gew.-Teilen eines paraffinischen Mineralöls.

Die neuen Verbindungen zeichnen sich durch eine hervorragende Wirksamkeit gegen ein breites Spektrum von pflanzenpathogenen Pilzen, insbesondere aus der Klasse der Deuteromyceten, Ascomyceten, Phycomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Rasen, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Schadpilze deren Lebensraum oder die von ihnen freizuhaltenden Pflanzen, Räume, Flächen oder Materialien mit einer wirksamen Menge der Wirkstoffe behandelt.

Die Anwendung erfolgt vor oder nach der Infektion der Materialien, Pflanzen oder Samen durch die Pilze.

Speziell eignen sich die neuen Verbindungen zur Bekämpfung folgender Pflanzenkrankheiten:
Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Zierpflanzen und Gemüse, Monilinia-Arten in Obst, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Alternaria-Arten an Gemüse und Obst.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,025 und 2, vorzugsweise 0,1 bis 1 kg Wirkstoff pro ha.

Bei der Saatgutbehandlung werden im allgemeinen Wirkstoffmengen von 0,001 bis 50, vorzugsweise 0,01 bis 10 g je Kilogramm Saatgut benötigt.

Die neuen Verbindungen können auch im Materialschutz (Holzschutz) eingesetzt werden, z.B. gegen Paecilomyces variotii.

Die erfindungsgemäßen Mittel können in der Anwendungsform als Fungizide auch zusammen mit anderen Wirkstoffen vorliegen, z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfid, Ammoniak-Komplex von Zink-(N,N-ethylen-bis-dithiocarbamat), Ammoniak-Komplex von Zink-(N,N'-propylen-bis-dithiocarbamat), Zink-(N,N'propylen-bis-dithiocarbamat), N,N'-Polypropylen-bis-(thiocarbamoyl)-disulfid;
Nitroderivate, wie Dinitro-(1-methylheptyl)-phenylcrotonat, 2-sec.-Butyl-4,6-dinitrophenyl-3,3-dimethylacrylat, 2-sec.-Butyl-4,6-dinitrophenyl-iso-propylcarbonat, 5-Nitro-iso-phthalsäure-di-iso-propylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4-triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid,
N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thion-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethylfuran-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan,
2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-2,2,4-triazol-1-yl)-2-butanon, (2-Chlorphenyl)-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol, [2-(4-Chlorphenyl)ethyl]-(1,1-dimethylethyl)-1H-1,2,4-triazol-1-ethanol, 1-[3-(2-Chlorphenyl)-1-(4-fluorphenyl)oxiran-2-yl-methyl]-1H-1,2,4-triazol sowie
verschiedene Fungizide, wie Dodecylguanidinacetat, 3-[3-(3,5-Dimethyl-2-oxycyclohexyl)-2-hydroxyethyl]glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methylester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[(3,5-Dichlorphenyl)-5-methyl-5-methoxymethyl-1,3-oxazolidin-2,4-dion, 3-(3,5-Dichlorphenyl)-1-iso-propylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol,
Strobilurine wie Methyl-E-methoximino-[α-(o-tolyloxy)-o-tolyl]acetat, Methyl-E-2-{2-[6-(2-cyanophenoxy)pyridimin-4-yloxy]phenyl}-3-methoxyacrylat, Methyl-E-methoximino-[α-(2,5-dimethyloxy)-o-tolyl]acetamid.

Anilino-Pyrimidine wie N-(4,6-dimethylpyrimidin-2-yl)anilin, N-[4-methyl-6-(1-propinyl)pyrimidin-2-yl]anilin, N-(4-methyl-6-cyclopropyl-pyrimidin-2-yl)anilin.

Phenylpyrrole wie 4-(2,2-difluor-1,3-benzodioxol-4-yl)pyrrol-3-carbonitril.

Zimtsäureamide wie 3-(4-chlorphenyl)-3-(3,4-dimethoxyphenyl)acrylsäuremorpholid.

### Synthesebeispiele

Die in den nachstehenden Synthesebeispielen wiedergegebenen Vorschriften zur Herstellung der Verbindungen I und III können unter Abwandlung der Ausgangsverbindungen zur Gewinnung weiterer Vertreter der allgemeinen Formeln I oder III benutzt werden. Die physikalischen Daten der demgemäß hergestellten Produkte sind in den anschließenden Tabellen 2 und 3 z.T. mit angegeben.

### Beispiel 1 (Vorprodukt vom Typ III)

### 2-Amino-5,4'-difluorbiphenyl (Nr. 2.3 in Tabelle 2)

Zu einer Lösung von 11,4 g (0,060 mol) 2-Brom-4-fluoranilin in 120 ml 1,2-Dimethoxyethan gab man unter Stickstoff 2,4 g Tetrakis-(triphenylphosphin)-palladium 15,1 g (0,108 mol) 4-Fluorphenylboronsäure und eine Lösung von 30 g (0,282 mol) Natriumcarbonat in 120 ml Wasser und erhitzte 8 Stunden am Rückfluß. Nach dem Abkühlen wurden 200 ml Methyl-tert.-butylether und 100 ml Wasser zugegeben. Die organische Phase wurde mit Wasser gewaschen, getrocknet und eingeengt. Nach Chromatographie des Rückstands an 50 g Kieselgel mit Cyclohexan als Laufmittel erhielt man 12,4 g der Titelverbindung (Fp: 67-69°C).

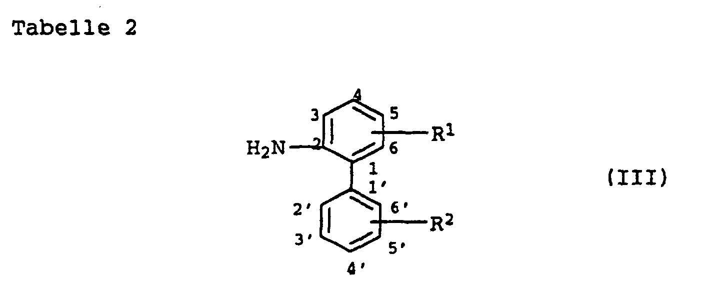

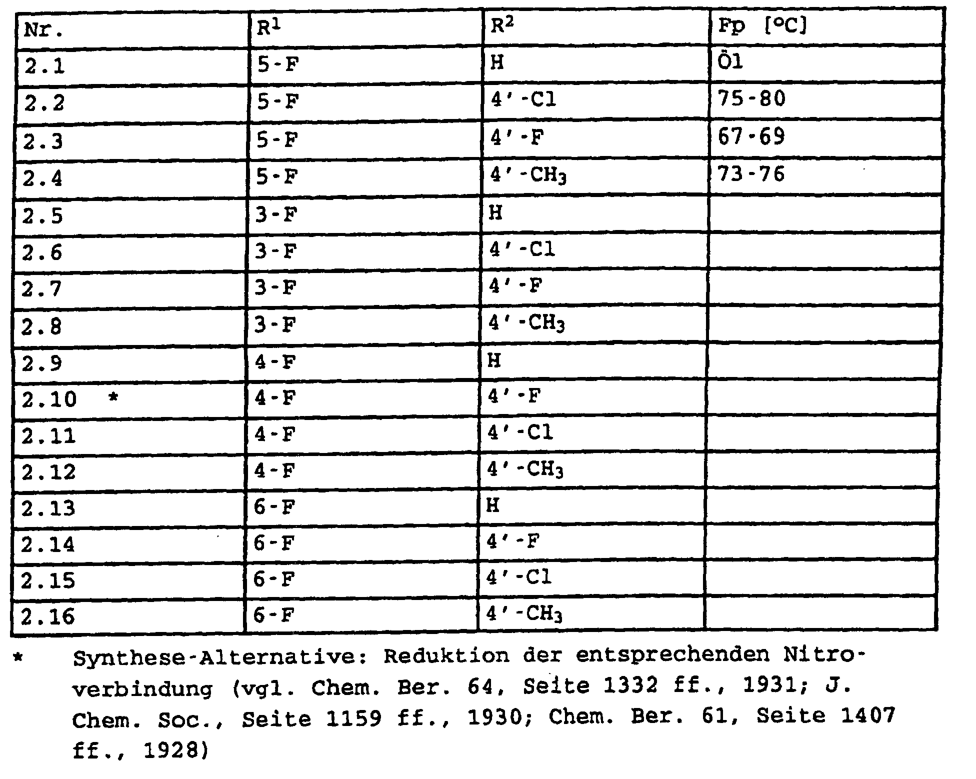

### Beispiel 2 (Wirkstoff vom Typ I)

### 1,3-Dimethyl-5-fluorpyrazol-A-carbonsäure-(4'-chlorbiphenyl)-amid

Zu einer Lösung von 1,42 g (7 mmol) 2-Amino-4'-chlor-biphenyl und 0,71 g (7 mmol) Triethylamin in 7 ml Tetrahydrofuran tropfte man bei + 5° C eine Lösung von 1,15 g (7 mmol) 1,3-Dimethyl-5-fluorpyrazol-4-carbonsäure-chlorid in 3 ml Tetrahydrofuran und rührte 20 min bei + 5°C und 2 Stunden bei Raumtemperatur nach. Nach dem Einrühren in 140 ml Wasser wurde der Niederschlag abgesaugt. Durch Anteigen mit einer Mischung aus Diisopropylether und Cyclohexan (1 : 2) erhielt man 1,5 g der Titelverbindung (Fp: 146-150°C. Nr. 1.10 in nachfolgender Tabelle).

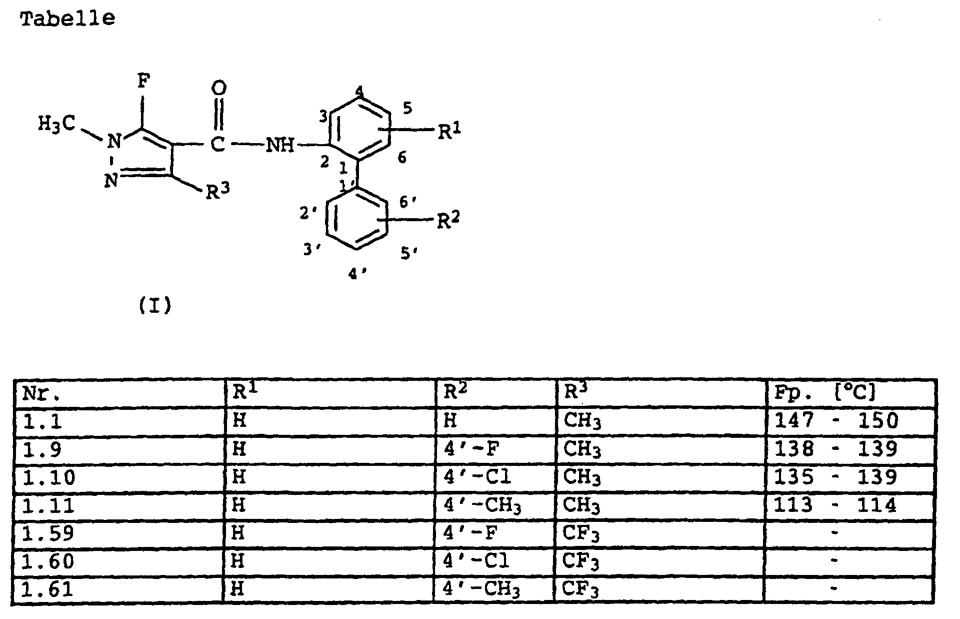

### Anwendungsbeispiele

Für die folgenden Versuche zur fungiziden Wirkung der Verbindungen I wurde eine Emulsion verwendet, welche zu 10 Gew.-% aus dem Wirkstoff und zu 90 Gew.-% aus einem Gemisch aus

| | |
|---|---|
| 70 Gew.-% | Cyclohexanol, |
| 20 Gew.-% | Nekanil® LN (Lutensol® AP6, Netzmittel mit Emulgier- und Dispergierwirkung auf der Basis ethoxylierter Alkylphenole) und |
| 10 Gew.-% | Emulphor® EL (Emulan® EL, Emulgator auf der Basis ethoxylierter Fettalkohole) |

bestand. Die gewünschten Wirkstoff-Konzentrationen wurden durch Verdünnen dieser Emulsion mit Wasser eingestellt.

Als Vergleichsverbindung "A" diente 2-Chlornicotinsäure-2'-ethylanilid, als Vergleichsverbindung "B" 2-Chlornicotinsäure-2'-phenylanilid. Beide Verbindungen sind bekannt aus der DE-A-24 17 216.

### Anwendungsbeispiel 1

### Botrytis cinerea

Scheiben von grünen Paprikaschoten wurden mit einer gemäß obiger Vorschrift hergestellten wäßrigen Aufbereitung tropfnaß gespritzt, welche jeweils 250 ppm eines einzigen Wirkstoffs enthielt. Als Wirkstoff wurde die erfindungsgemäße Verbindung 1.10 verwendet.

2 Stunden nach dem Antrocknen des Spritzbelags wurden die Fruchtscheiben mit einer Sporensuspension des Pilzes Botrytis cinerea, welche 1,7 x 10⁶ Sporen pro ml einer 2%-igen Biomalzlösung enthielt, inokuliert. Die Fruchtscheiben wurden anschließend 4 Tage bei 18°C in Kammern hoher Luftfeuchtigkeit inkubiert.

Die visuelle Bewertung ergab für die genannte Verbindung einen Pilzbefall von 0-15% der Scheibenoberflächen.

Im Falle der Verbindung "A" lag der Pilzbefall unter ansonsten gleichen Versuchsbedingungen bei 100 %.

Scheiben, welche nicht mit einer Verbindung I oder der Verbindung "A" behandelt worden waren, wiesen einen Befall von 100 % auf.

### Anwendungsbeispiel 2

### Erysiphe graminis var. tritici

Blätter von in Töpfen gewachsenen Weizenkeimlingen (Sorte "Frühgold") wurden mit einer gemäß obiger Vorschrift hergestellten wäßrigen Aufbereitung besprüht, welche jeweils 250 ppm eines einzigen Wirkstoffs enthielt. Als Wirkstoff wurde die erfindungsgemäße Verbindung 1.10 verwendet.

24 Stunden nach dem Antrocknen des Spritzbelags wurden die Blätter mit Oidien (Sporen) des Weizenmehltaus (Erysiphe graminis var. tritici) bestäubt. Die Pflanzen wurden anschließend für 7 Tage bei 20-22°C und einer relativen Luftfeuchtigkeit von 75-80% inkubiert.

Die visuelle Bewertung ergab für die genannte Verbindung einen Pilzbefall von 5-25% der Blattoberfläche.

Im Falle der Verbindung "A" lag der Pilzbefall unter ansonsten gleichen Versuchsbedingungen bei 60 %. Für "B" wurde ein Befall von 80 % ermittelt.

Blätter welche nicht mit einer Verbindung I, "A" oder "B" behandelt worden waren, wiesen einen Befall von 80% auf.

## Patentansprüche

1. Biphenylamide der allgemeinen Formel I 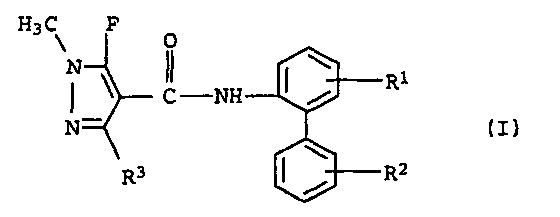 sowie deren Salze, in der die Reste R¹, R² und R³ die folgenden Bedeutungen haben:
R¹ Wasserstoff oder Fluor;
R² Wasserstoff, Halogen, C₁-C₄-Alkyl, Halogenmethyl, C₁-C₄-Alkoxy oder C₁-C₄-Alkylthio;
R³ Methyl, Difluormethyl oder Trifluormethyl.

2. Verfahren zur Herstellung von Biphenylamiden der allgemeinen Formel I gemäß Anspruch 1, **dadurch gekennzeichnet, daß** man ein Carbonsäurehalogenid der allgemeinen Formel II 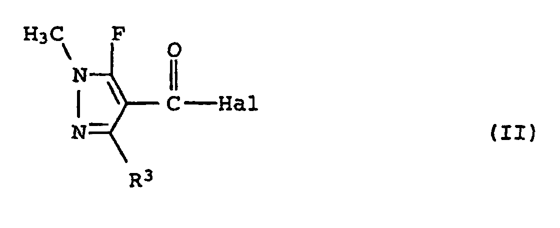 in der Hal für Halogen steht, mit einem Biphenylamin der allgemeinen Formel III 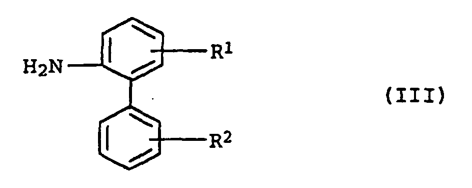 unter Zuhilfenahme einer Base umsetzt.

3. Zur Bekämpfung von Schadpilzen geeignete Mittel, enthaltend eine wirksame Menge mindestens einer Verbindung der allgemeinen Formel I oder eines ihrer Salze gemäß Anspruch 1 und mindestens ein übliches Formulierungshilfsmittel.

4. Verfahren zur Herstellung der Mittel gemäß Anspruch 3, **dadurch gekennzeichnet, daß** man eine fungizid wirksame Menge mindestens einer Verbindung der allgemeinen Formel I oder einem ihrer Salze gemäß Anspruch 1 mit mindestens einem üblichen Formulierungshilfsmittel in an sich bekannter Weise miteinander verarbeitet.

5. Verfahren zur Bekämpfung von Schadpilzen, **dadurch gekennzeichnet, daß** man die Schadpilze, deren Lebensraum oder die von ihnen freizuhaltenden Pflanzen, Räume, Flächen oder Materialien mit einer wirksamen Menge mindestens einer Verbindung der allgemeinen Formel I oder einem ihrer Salze gemäß Anspruch 1 oder mit einem I oder einem ihrer Salze enthaltenden Mittel gemäß Anspruch 3 behandelt.

6. Verwendung der Verbindungen der allgemeinen Formel I oder ihrer Salze gemäß Anspruch 1 oder der Mittel gemäß Anspruch 3 zur Bekämpfung von Schadpilzen.

## Claims

1. A biphenylamide of the formula I 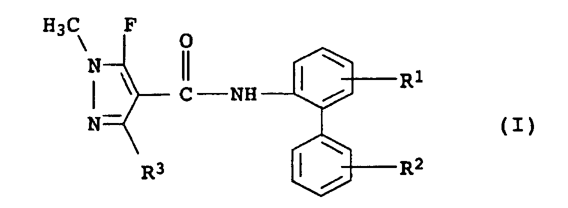 and salts thereof, where the radicals R¹, R² and R³ have the following meanings:
R¹ is hydrogen or fluorine;
R² is hydrogen, halogen, C₁-C₄-alkyl, halomethyl, C₁-C₄-alkoxy or C₁-C₄-alkylthio;
R³ is methyl, difluoromethyl or trifluoromethyl.

2. A process for preparing biphenylamides of the formula I as claimed in claim 1, which comprises reacting an acyl halide of the formula II 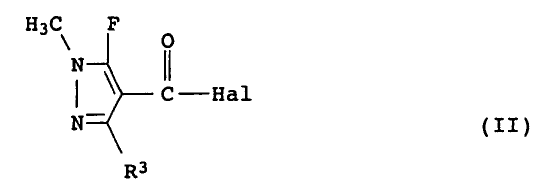 in which Hal is halogen, with a biphenylamine of the formula III 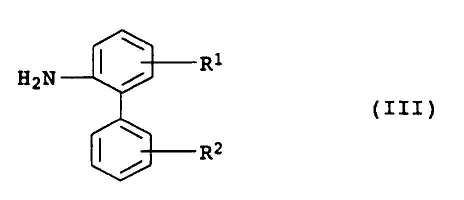 with the aid of a base.

3. A composition suitable for controlling harmful fungi, which comprises an effective amount of at least one compound of the formula I or a salt thereof as claimed in claim 1 and at least one customary formulation auxiliary.

4. A process for preparing the composition as claimed in claim 3, which comprises jointly processing, in a manner known per se, a fungicidally effective amount of at least one compound of the formula I or a salt thereof as claimed in claim 1 with at least one customary formulation auxiliary.

5. A method for controlling harmful fungi, which comprises treating the harmful fungi, their habitat or the plants, spaces, areas or materials to be kept free from them with an effective amount of at least one compound of the formula I or a salt thereof as claimed in claim 1 or with a composition comprising I or a salt thereof as claimed in claim 3.

6. The use of the compound of the formula I or a salt thereof as claimed in claim 1 or of the composition as claimed in claim 3 for controlling harmful fungi.

## Revendications

1. Biphénylamides de la formule générale I : 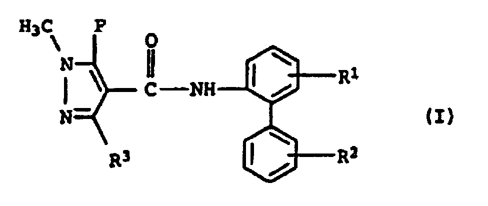 dans laquelle les radicaux R¹, R² et R³ ont les significations suivantes :
R¹ est de l'hydrogène ou du fluor,
R² est de l'hydrogène, un halogène, un radical alkyle en C₁-C₄, halogénométhyle, alcoxy en C₁-C₄ ou alkylthio en C₁-C₄,
R³ est un radical méthyle, difluorométhyle ou trifluorométhyle,
ainsi que leurs sels.

2. Procédé de préparation de biphénylamides de la formule générale I suivant la revendication 1, **caractérisé en ce qu'**on fait réagir un halogénure d'acide carboxylique de la formule générale II : 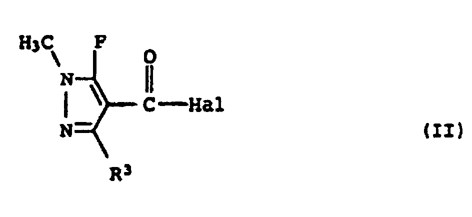 dans laquelle Hal représente un halogène, avec une biphénylamine de la formule générale III : 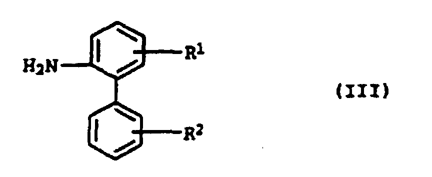 avec l'aide d'une base.

3. Produits appropriés pour lutter contre des champignons nuisibles, contenant une quantité efficace d'au moins un composé de la formule générale I ou d'un de ses sels suivant la revendication 1 et au moins un adjuvant de formulation courant.

4. Procédé de préparation des produits suivant la revendication 3, **caractérisé en ce que**, d'une manière connue en soi, on traite mutuellement une quantité efficace du point de vue fongicide d'au moins un composé de la formule générale I ou d'un de ses sels suivant la revendication 1 par au moins un adjuvant de formulation courant.

5. Procédé de lutte contre des champignons nuisibles, **caractérisé en ce qu'**on traite les champignons nuisibles, leur biotope ou les plantes, espaces, surfaces ou matières à libérer de ceux-ci par une quantité efficace d'au moins un composé de la formule générale I ou d'un de ses sels suivant la revendication 1 ou par un produit contenant un composé I ou un de ses sels suivant la revendication 3.

6. Utilisation des composés de la formule générale I ou de leurs sels suivant la revendication 1 ou des produits suivant la revendication 3 pour lutter contre des champignons nuisibles.
